(19) Europäisches Patentamt
European Patent Office
Office européen des brevets

(11) **EP 4 653 489 A1**

(12) **EUROPEAN PATENT APPLICATION**
published in accordance with Art. 153(4) EPC

(43) Date of publication:
**26.11.2025 Bulletin 2025/48**

(21) Application number: **24744894.7**

(22) Date of filing: **18.01.2024**

(51) International Patent Classification (IPC):
***C08J 3/24*** (2006.01) ***C08J 3/12*** (2006.01)
***C08B 15/00*** (2006.01) ***C08L 1/28*** (2006.01)
***C08K 5/00*** (2006.01) ***C08K 5/092*** (2006.01)
***C08K 5/109*** (2006.01) ***A61L 15/22*** (2006.01)

(52) Cooperative Patent Classification (CPC):
**A61L 15/22; C08B 15/00; C08J 3/12; C08J 3/24; C08K 5/00; C08K 5/092; C08K 5/109; C08L 1/28**

(86) International application number:
**PCT/KR2024/000880**

(87) International publication number:
**WO 2024/155114 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.01.2023 KR 20230008025**

(71) Applicant: **LG CHEM, LTD.**
**Seoul 07336 (KR)**

(72) Inventors:
- **KIM, Yu Min**
  **Daejeon 34122 (KR)**
- **CHO, Beom Shin**
  **Daejeon 34122 (KR)**
- **KANG, Sun Ah**
  **Daejeon 34122 (KR)**
- **YUN, Hae Sung**
  **Daejeon 34122 (KR)**

(74) Representative: **Plasseraud IP**
**104 Rue de Richelieu**
**CS92104**
**75080 Paris Cedex 02 (FR)**

(54) **POLYMER MATERIAL**

(57) The present application can provide a polymer material, a preparation method therefor, and a use thereof. The present application can provide a polymer material made of a material with biodegradability and exhibiting balanced absorption properties. The present application can also provide a preparation method for such a polymer material, and a use thereof.



Processed by Luminess, 75001 PARIS (FR)

## Description

### Technical Field

This application claims the benefit of priority based on Korean Patent Application No. 10-2023-0008025 dated January 19, 2023, the disclosure of which is incorporated herein by reference in its entirety.

The present application relates to a polymer material and a use thereof.

### Background Art

A hydrogel polymer or hydrogel is generally defined as a cross-linked hydrophilic polymer.

Such a polymer can be used as a material called an SAP (Super Absorbent Polymer). The SAP is a material that can absorb moisture tens to thousands of times its own weight. The SAP is used for various applications, such as sanitary products such as hygiene products or diapers, medical products, household materials, agricultural materials, horticultural materials, transportation materials, civil engineering and construction materials, materials related to electrical and electronic devices, or water treatment agents.

The most widely used hydrogel polymer, which is used as the SAP, is a polymer made of a vinyl-based material such as cross-linked polyacrylic acid.

Such materials are relatively inexpensive and have excellent water absorption capacity, but cause various problems because they remain semi-permanently even after disposal.

To solve such problems, there are various attempts to manufacture the SAP from so-called materials with biodegradability.

However, the materials known to date do not form the SAP with balanced physical properties. For example, the most representative physical property required for the SAP is absorption capacity, but in the SAP of a biodegradable material known to date, at least one characteristic of absorption capacity and biodegradability is not satisfactorily secured, or in some cases, both physical properties are not secured at an appropriate level.

Absorption capacity is usually evaluated through CRC (centrifuge retention capacity), AUP (absorption capacity under pressure), and a vortex test, and the like. In the CRC evaluation, the retention capacity of the polymer material is evaluated in a state where no pressure is applied, and in the AUP evaluation, the absorption characteristics are evaluated in a state where a certain level of pressure is applied to the polymer material. However, even in the case of materials with excellent CRC characteristics, there are cases where AUP characteristics are not secured due to poor absorption characteristics under pressure.

Accordingly, to improve the AUP characteristics of absorbent materials, the gel strength is often strengthened by introducing a cross-linking agent on the surface of the absorbent material, but in this case, the absorption time is reduced, so that there is a problem that the vortex test evaluation results are poor. Therefore, it is a difficult problem to obtain a material ensuring balanced CRC, AUP, and vortex test results corresponding to absorption characteristics.

### Disclosure

### Technical Problem

The present application relates to a polymer material, a preparation method therefor, and a use thereof. The present application is intended to provide a polymer material made of a material with biodegradability and exhibiting balanced absorption properties. The present application is also intended to provide a preparation method for such a polymer material, and a use thereof.

### Technical Solution

Among the physical properties mentioned in this specification, when the measurement temperature and/or pressure affects the physical property value, the relevant physical property means a physical property measured at room temperature and/or normal pressure, unless specifically mentioned otherwise.

In the present application, the term room temperature is a natural temperature without heating or cooling, which may mean, for example, any one temperature in a range of about 10°C to 30°C, or a temperature of 23°C or 25°C or so.

In the present application, the term normal pressure is a pressure when not particularly reduced or increased, which may mean a pressure of atmospheric pressure or so, for example, about 740 mmHg to 780 mmHg or so.

Among the physical properties mentioned in this specification, when the measurement humidity affects the physical property value, the physical property means a physical property measured at natural humidity without special adjustment at the measured temperature and pressure, unless otherwise specified.

In this specification, unless otherwise specified, the term alkyl or alkyl group means an alkyl or alkyl group with 1 to 20 carbon atoms, 1 to 16 carbon atoms, 1 to 12 carbon atoms, 1 to 8 carbon atoms, or 1 to 4 carbon atoms. Such an alkyl or alkyl group may be linear, branched, or cyclic. Such an alkyl or alkyl group may also be optionally substituted with one or more substituents.

In this specification, unless otherwise specified, the term alkylene or alkylene group means a functional group which is connected to another object by separating two hydrogen atoms from an alkane, wherein it has a structure that the two hydrogen atoms are separated from other carbon atoms of the alkane. Such an alkylene or alkylene group may be an alkylene or alkylene group with 2 to 20 carbon atoms, 2 to 16 carbon atoms, 2 to 12 carbon atoms, 2 to 8 carbon atoms, or 2 to 4 carbon atoms. Such an alkylene or alkylene group may be linear, branched, or cyclic. Such an alkylene or alkylene group may also be optionally substituted with one or more substituents.

In this specification, unless otherwise specified, the term alkylidene or alkylidene group means a functional group which is connected to another object by separating two hydrogen atoms from an alkane, wherein it has a structure that the two hydrogen atoms are separated from one carbon atom of the alkane. Such an alkylidene or alkylidene group may be an alkylidene or alkylidene group with 1 to 20 carbon atoms, 1 to 16 carbon atoms, 1 to 12 carbon atoms, 1 to 8 carbon atoms, or 1 to 4 carbon atoms. Such an alkylidene or alkylidene group may be linear, branched, or cyclic. Such an alkylidene or alkylidene group may also be optionally substituted with one or more substituents.

In the present application, the term hydrogel polymer material means an absorbent material comprising a cross-linked polymer. In this specification, the material may also be referred to as a hydrogel.

The polymer material of the present application may be the hydrogel. The hydrogel of the present application may have balanced absorption characteristics, and may exhibit, particularly, a rapid absorption time while exhibiting appropriate levels of centrifuge retention capacity (CRC) and absorption capacity under pressure (AUP).

For example, the polymer material may have a vortex absorption time for a 0.9 wt% NaCl aqueous solution within a predetermined range. The vortex absorption time means a rate of absorbing the 0.9 wt% NaCl aqueous solution measured in the manner described in Example sections of this specification. The upper limit of the vortex absorption time of the polymer material may be 150 seconds, 140 seconds, 130 seconds, 120 seconds, 110 seconds, 100 seconds, 90 seconds, 80 seconds, 70 seconds, or 65 seconds or so. Since the shorter the absorption time means that it has the faster absorption rate, the lower limit thereof is not particularly limited, and for example, the lower limit of the absorption time may be 0 seconds, 10 seconds, 20 seconds, 30 seconds, 40 seconds, 50 seconds, 60 seconds, or 65 seconds or so. The absorption time may be less than any one of the above-described upper limits, or within a range of less than or equal to, or less than any one of the above-described upper limits while being more than or equal to, or more than any one of the above-described lower limits.

The lower limit of the centrifuge retention capacity (CRC) of the polymer material according to EDANA (European Disposables and Nonwovens Association) method WSP 241.3 may be 10 g/g, 15 g/g, 20 g/g, 25 g/g, 30 g/g, 35 g/g, 40 g/g, or 45 g/g or so, and the upper limit thereof may be 60 g/g, 55 g/ g, 50 *g/g,* 45 g/g, 40 g/g, or 35 g/g or so. The centrifuge retention capacity (CRC) may be more than or equal to, or more than any one of the above-described lower limits, or within a range of less than or equal to, or less than any one of the above-described upper limits while being more than or equal to, or more than any one of the above-described lower limits.

For example, the lower limit of absorption capacity under pressure (AUP) of the polymer material at 0.7 psi according to EDANA (European Disposables and Nonwovens Association) method WSP 242.3 may be 1.5 *g/g,* 2 g/g, 2.5 g/g, 3 g/g, 3.5 g/g, 4 g/g, 4.5 g/g, *5 g/g,* 5.5 g/g, 6 g/g, 6.5 g/g, 7 g/g, 7.5 g/g, 8 g/g, 8.5 g/g, 9 g/g, 9.5 g/g, 10 g/g, 10.5 *g/g,* 11 g/g, or 11.5 *g/g* or so, and the upper limit thereof may be 40 g/g, 35 g/g, 30 g/g, 20 *g/g, 15 g/g,* 10 g/g, 8 g/g, 6 g/g, or 4 g/g or so. The absorption capacity (AUP) may be more than or equal to, or more than any one of the above-described lower limits, or within a range of less than or equal to, or less than any one of the above-described upper limits while being more than or equal to, or more than any one of the above-described lower limits.

The polymer material may exhibit at least one of the above-described centrifuge retention capacity and absorption capacity under pressure while exhibiting the above-described vortex absorption time, or may exhibit the above-described centrifuge retention capacity and absorption capacity under pressure while exhibiting the above-described vortex absorption time.

The polymer material of the present application is made of a biodegradable material as its main component, which may exhibit excellent biodegradability.

For example, the lower limit of the biodegradability degree of the polymer material may be 50%, 52%, 54%, 56%, 58%, 60%, 62%, 64%, 66%, 68%, 70%, 72%, 74%, 76%, 78%, 80%, 82%, 84%, 86%, 88%, 90%, 92%, 94%, 96%, 98%, or 99% or so, and the upper limit thereof may be 100%, 98%, 96%, 94%, 92%, 90%, 88%, 86%, 84%, 82%, 80%, 78%, or 76% or so. The biodegradability degree may be more than or equal to, or more than any one of the above-described lower limits, or within a range of less than or equal to, or less than any one of the above-described upper limits while being more than or equal to, or more than any one of the above-described lower limits. The biodegradability degree is measured in the manner described in the examples of this specification.

The present application relates to a polymer material.

The term polymer material means a material comprising a polymer. Here, the polymer may mean a substance formed by connecting two or more unitary bodies by covalent bonds. In one example, the polymer may mean a substance comprising a structure in which two or more unitary bodies are connected by covalent bonds, and having a molecular weight of a certain level or more. The range of the molecular weight is not limited. In one example, the lower limit of the molecular weight of the polymer may be, in terms of weight average molecular weight (Mw), 500 g/mol, 1,000 g/mol, 10,000 g/mol, 100,000 g/mol, 1,000,000 g/mol, or 10,000,000 g/mol or so, and the upper limit thereof may be 10,000,000,000 g/mol, 1,000,000,000 g/mol, 100,000,000 g/mol, or 10,000,000 g/mol or so. The weight average molecular weight may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

The weight average molecular weight is a value measured in the manner described in the examples of this specification.

In one example, the lower limit of the polymer content in the polymer material may be 55 wt%, 60 wt%, 65 wt%, 70 wt%, 75 wt%, 80 wt%, 85 wt%, 90 wt%, or 95 wt% or so, and the upper limit thereof may be 100 wt%, 98 wt%, 96 wt%, 94 wt%, 92 wt%, or 90 wt% or so. The ratio may be more than or equal to, or more than any one of the above-described lower limits, or within a range of less than or equal to, or less than any one of the above-described upper limits while being more than or equal to, or more than any one of the above-described lower limits.

The polymer material of the present application may comprise a polysaccharide component.

The term polysaccharide component means a polysaccharide or a mixture of polysaccharides. In the case of a mixture of polysaccharides, the mixture may be a mixture of one type of polysaccharide (i.e., when two or more molecules of the same type of polysaccharide are present) or a mixture of two or more types of polysaccharides. Here, two or more types of polysaccharides may also mean different types of polysaccharides, and may also include polysaccharides which have the same type, but different physical properties such as a molecular weight. The polysaccharide component includes only polysaccharides.

The term polysaccharide has a meaning known in the industry. The polysaccharide generally refers to a polymer molecule in which two or more unitary bodies are linked by covalent bonds. The covalent bond connecting the unitary bodies is usually a glycosidic bond. Typically, a structure in which two unitary bodies are linked by a covalent bond such as a glycosidic bond is called a disaccharide. In this specification, unless otherwise specified, the disaccharide is also included in the category of polysaccharide.

The unitary body forming the polysaccharide may be a biomolecule composed of carbon, hydrogen, and oxygen, or composed of carbon, hydrogen, oxygen, and nitrogen. In this specification, the term biomolecule is interpreted to have the meaning generally applied in the industry. Typically, as an example of the biomolecule in the industry, monosaccharides such as glucose, galactose, fructose or xylose, disaccharides such as sucrose, lactose, maltose or trehalose, polyols such as sorbitol or mannitol, oligosaccharides such as maltodextrin, dextrin, raffinose, stachyose or fructo-oligosaccharide and/or amino sugars such as glucosamine or N-acetal glucosamine, and the like are known, but the types of biomolecules in the present application are not limited to the foregoing.

If the polymer (e.g., polysaccharide component) contained in the polymer material is in a cross-linked state and has absorption capacity, the relevant polymer material may be called the hydrogel polymer material or hydrogel herein. In one example, the polymer material may also be in a powder state formed through a grinding process or the like.

In the polymer material of the present application, the polysaccharide component may be in a cross-linked state. Here, the cross-linking means a state where two or more molecules of polysaccharide are connected by one or more chemical bonds. The cross-linking may also be formed by a chemical substance, which is referred to as a so-called cross-linking agent, other than the polysaccharide, and may be formed by a reaction between functional groups included in the polysaccharide. In this specification, when the cross-linking of the polysaccharide is performed by the reaction between functional groups included in the polysaccharide without applying other cross-linking agents, the relevant cross-linked polysaccharide may be called a self-cross-linked polysaccharide component.

In this specification, the polymer material may comprise at least a self-cross-linked polysaccharide component among the above types of cross-linked polysaccharides.

In one example, the polymer material may comprise the self-cross-linked polysaccharide component in a certain amount or more based on the total weight of the polymer material. For example, the lower limit of the ratio of the self-cross-linked polysaccharide component in the polymer material may be 55 wt%, 60 wt%, 65 wt%, 70 wt%, 75 wt%, 80 wt%, 85 wt%, 90 wt%, or 95 wt% or so, and the upper limit thereof may be 100 wt%, 98 wt%, 96 wt%, 94 wt%, 92 wt%, or 90 wt% or so. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits.

In another example, the ratio of the cross-linking agent cross-linking the polysaccharide component in the polymer material may be limited to a certain amount or less. Here, the cross-linking agent is a substance to form a chemical

bond connecting the polysaccharide components, which means a different substance other than the polysaccharide. For example, the upper limit of the ratio of the cross-linking agent in the polymer material may be 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof may be 0 wt% or so. The ratio of the cross-linking agent may be less than any one of the above-described upper limits, or within a range of less than or equal to, or less than any one of the above-described upper limits while being more than or equal to, or more than any one of the above-described lower limits.

In the present application, the cross-linking of the polysaccharide is performed without using a cross-linking agent or while minimizing the used amount of cross-linking agent. If a cross-linking agent other than polysaccharides is applied, the biodegradability of the polymer material may be reduced. However, since the cross-linking efficiency of the polysaccharide is generally lowered when a cross-linking agent is not used, it is not easy to obtain a polymer material with desired properties (e.g., absorption properties). In the present application, by applying a polysaccharide having one or more characteristics among the polysaccharides to be described below and/or applying a self-cross-linking reaction in a manner to be described below, it is possible to provide a new polymer material having balanced absorption capacity while having excellent biodegradation characteristics in a self-cross-linked state.

In the present application, the self-cross-linking of the polysaccharide may be performed using a so-called acidic polysaccharide. As is known, the acidic polysaccharide is a polysaccharide having this acidic group, where an example of the acidic group may include a carboxylic group, a phosphate group, a phosphite group, and/or a sulfuric acid ester group (sulfuric ester group), or their salts.

A substitution degree of the acidic polysaccharide or acidic polysaccharide component may be adjusted for appropriate self-cross-linking. The substitution degree is an indicator indicating the amount of the acidic group present in the acidic polysaccharide or polysaccharide component, where such a substitution degree may be, for example, a value obtained before the polysaccharide or polysaccharide component implements the cross-linked structure.

For example, the lower limit of the substitution degree may be 0.1, 0.15, 0.2, 0.25, 0.3, 0.35, 0.4, 0.45, 0.5, 0.6, 0.65, 0.7, 0.75, 0.8, 0.85, 0.9, 0.95, or 1 or so, and the upper limit thereof may be 2.5, 2, 1.5, 1.1, 1.05, 1, 0.95, 0.9, 0.85, 0.8, 0.75, 0.7, 0.65, or 0.6 or so. The substitution degree may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. Within the range of the substitution degree, the cross-linking of the polysaccharide may proceed effectively, and the physical properties (for example, absorption capacity and/or biodegradability, etc.) of the resulting polymer material may be stably secured. When the substitution degree is high, the number of acidic groups, which are hydrophilic functional groups, increases in the polysaccharide or polysaccharide component, so that it may be advantageous in terms of absorption characteristics, but too many acidic groups excessively promote the cross-linking reaction, whereby absorption characteristics after cross-linking may be lowered. Therefore, considering these points, it is possible to select an appropriate substitution degree.

In this specification, the substitution degree is an indicator of the degree of acidic group present in the polysaccharide or polysaccharide component, which is, for example, a value representing to what extent the functional group such as a hydroxy group present in each unitary body contained in the polysaccharide or polysaccharide component is substituted with a predetermined acidic group (for example, the carboxyl group) and is an average value for each unitary body present in the polysaccharide. For example, if the unitary body is a grape sugar (glucose) unit, there are three hydroxy groups in the relevant unit before modification, and thus, if all the hydroxy groups are replaced with the functional groups of Formula 1 above, the substitution degree for the relevant unit is 3. However, the substitution degree of the polysaccharide is the average value of the substitution degree of each unitary body present in the relevant polysaccharide, so that for example, in a polysaccharide containing 5 grape sugar (glucose) units, if the substitution degree of each unit is 1, 0, 2, 3, and 1, the substitution degree of the polysaccharide becomes 1.4, which is the average value. This substitution degree may be identified through $^1$H NMR analysis of the polysaccharide. That is, since the hydroxyl groups and substituted functional groups present in the polysaccharide can be quantified through the $^1$H NMR analysis, the substitution degree can be identified, and the substitution degree can be calculated in consideration of the $^1$H NMR analysis results of the polysaccharide before modification, if necessary. In this way, the method of quantifying functional groups through $^1$H NMR analysis is known.

In the present application, the self-cross-linking of the polysaccharide may be performed, for example, using a polysaccharide or polysaccharide component having a carboxyl group as an acidic group among such acidic polysaccharides or acidic polysaccharide components. Since the polysaccharide itself contains hydroxy groups, the hydroxy groups contained in one molecule of polysaccharide may be subjected to an esterification reaction with the carboxyl groups contained in the acidic polysaccharide to perform the self-cross-linking.

The type of polysaccharide component having the carboxyl group is not particularly limited. For example, the self-cross-linking may be performed by applying polysaccharides having carboxyl groups on their own, such as so-called CMC (carboxylmethyl cellulose), which corresponds to a lignocellulosic polysaccharide, or polysaccharides to which carboxyl groups are introduced through processes such as maleation or carboxyalkylation.

The self-cross-linked polysaccharide component may comprise polymer chains (i.e., polysaccharide chains to be cross-linked) containing monosaccharide units linked by glycosidic bonds, and cross-linking bonds linking the polymer chains.

At this time, the cross-linking bond may be connected to the monosaccharide unit.

Here, the cross-linking bond may be a bond represented by Formula 1 below.

[Formula 1]

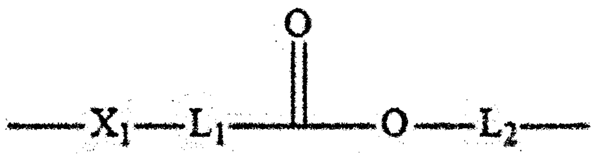

In Formula 1, $X_1$ is an oxygen atom or $NR_{11}$, where $R_{11}$ is a hydrogen atom, an alkyl group or an alkylcarbonyl group, and $L_1$ is an alkylene group, an alkylidene group, or a bond of Formula 2 below, and $L_2$ is represented by a single bond or $-(CH_2)-O-$.

[Formula 2]

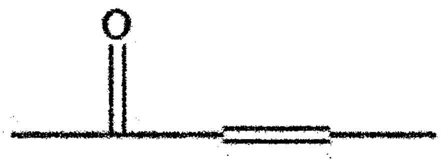

In Formula 1, $X_1$ shown on the leftmost side may be directly connected to the monosaccharide unit of the polymer chain.

In the case where $X_1$ of Formula 1 is an oxygen atom, it is the case where starch or the like as the polysaccharide is applied to the cross-linking, and in the case of $NR_{11}$, it is the case where chitosan or chitin, and the like is applied to the cross-linking.

In Formula 1, when $L_2$ is a single bond, the $L_2$ does not exist. That is, in Formula 1, when $L_2$ exists, $L_2$ may be directly connected to the monosaccharide unit of the polymer chain, and when $L_2$ is a single bond, the oxygen atom on the left side of $L_2$ in Formula 1 may be directly connected to the monosaccharide unit.

In the case where the self-cross-linking of the polysaccharide is performed by a polysaccharide having a carboxyl group on its own, such as CMC (carboxylmethyl cellulose), or a polysaccharide having a carboxyl group introduced by carboxyalkylation or the like, $L_1$ in Formula 1 may usually be an alkylene group or an alkylidene group. In Formula 1, when $L_1$ is a functional group of Formula 2, it is the case where the self-cross-linking is performed by a carboxyl group introduced by so-called maleation or the like.

In Formula 1, when $L_1$ is Formula 2, the carbon atom of the carbonyl group in Formula 2 is connected to $X_1$ on the left side of $L_1$ in Formula 1, and the carbon atom on the right side of the carbon-carbon double bond in Formula 2 is connected to the carbon atom of the carbonyl group on the right side of $L_1$ in Formula 1 above.

One or more bonds of Formula 1 above may exist within the self-cross-linked polysaccharide component.

The type of the monosaccharide unit is not particularly limited, which may be typically a monosaccharide unit constituting a polysaccharide. For example, such a monosaccharide unit may be a glucose unit, a glucosamine unit, an N-acetylglucosamine unit, and the like.

Such a monosaccharide unit usually contains a ring structure containing carbon atoms and oxygen atoms as ring constituent atoms. The ring structure of the monosaccharide unit is usually a hexa-cyclic ring structure (in the case where the ring atoms include only 5 carbon atoms and 1 oxygen atom), but may also have a ring structure of 6 rings or more. When the ring structure is six rings or more, the ring atoms may be carbon atoms, or heteroatoms such as oxygen or nitrogen atoms.

In the case of the self-cross-linking structure, the bond of Formula 1 above may be directly connected to the carbon atom of the ring structure of the monosaccharide unit, or may be connected via a methylene group ($-CH_2-$).

At least one of or both the leftmost oxygen atom and the rightmost oxygen atom (if $L_2$ is a single bond, it means the oxygen atom connected to the right side of the carbonyl group, and if it is not a single bond, it means the oxygen atom of $-(CH_2)-O-$) in Formula 1 may be directly connected to the carbon atom of the ring structure, or may be connected via the methylene group ($-CH_2-$).

Here, the matter of being connected via the methylene group ($-CH_2-$) is the case where only the methylene group ($-CH_2-$) exists between the leftmost oxygen atom of Formula 1 above or the rightmost oxygen atom (if $L_2$ is a single bond, it means the oxygen atom connected to the right side of the carbonyl group, and if it is not a single bond, it means the oxygen atom of $-(CH_2)-O-$) of Formula 1 and the carbon atom of the ring structure.

In this case, more specifically, the polysaccharide component may contain a unit represented by Formula 3 below.

[Formula 3]

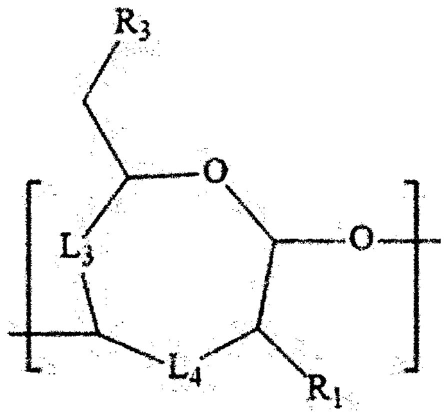

In Formula 3, $R_1$ is a hydroxy group, an amino group, $-L_5-C(=O)-OH$, $-L_5-C(=O)-O-$, or a functional group of Formula 4 below, $R_3$ is a hydroxy group, $-L_5-C(=O\ )-OH$, $-L_5-C(=O)-O-$ or a functional group of Formula 4 below, and one of $L_3$ and $L_4$ is a single bond, the other is $CHR_2$, where $R_2$ is a hydroxy group, $-L_5-C\ (\ =O)-OH$, $-L_5-C(=O)-O-$ or a functional group of Formula 4 below, and $L_5$ is an alkylene group or an alkylidene group, but any one of $R_1$ to $R_3$ is the oxygen atom (excluding the oxygen atom present in the carbonyl group) of the bond of Formula 1 above.

[Formula 4]

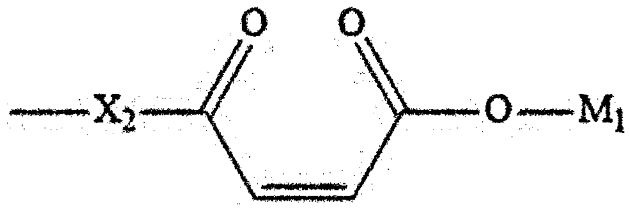

In Formula 4, $X_2$ is an oxygen atom or $NR_{11}$, where $R_{11}$ is a hydrogen atom, an alkyl group or an alkylcarbonyl group, $M_1$ is hydrogen or a metal, and when $M_1$ is the metal, the bond of $O-M_1$ above is an ionic bond.

The functional group of $-L_5-C(=O)-OH$ or $-L_5-C(=O)-O-$ in Formula 3 may be, for example, a carboxyl group introduced by carboxyalkylation, or a functional group in which the carboxyl group is ionized, and the like, and Formula 4 is a functional group introduced by maleation.

Such functional groups are introduced for forming cross-linking bonds by participating in the above-described self-cross-linking reaction, but all the introduced functional groups may not participate in the cross-linking reaction, and in this case, some functional groups may remain.

Here, the fact that any one of $L_3$ and $L_4$ is a single bond means that any one of $L_3$ and $L_4$ does not exist. For example, when $L_3$ is not present, the carbon atoms connected to the left and right of $L_3$ in Formula 3 are directly connected, and when $L_4$ is not present, the carbon atoms connected to the left and right of $L_4$ in Formula 3 are directly connected.

The fact that the other of $L_3$ and $L_4$ above is $CHR_2$ means that in Formula 3, any one of $L_3$ and $L_4$ is a carbon atom, and the substituent $R_2$ is substituted on the carbon atom.

The fact that in Formula 3, any one of $R_1$ to $R_3$ is the oxygen atom (excluding the oxygen atom present in the carbonyl group) of the bond of Formula 1 means that any one of $R_1$ to $R_3$ is the oxygen atom of the bond of Formula 1 connecting the polysaccharide, where the oxygen atom means any one of the leftmost oxygen atom and the rightmost oxygen atom in Formula 1 (if $L_2$ is a single bond, it means the oxygen atom connected to the right of the carbonyl group, and if it is not a single bond, it means the oxygen atom of $-(CH_2)-O-$).

In the case where $X_2$ of Formula 4 is an oxygen atom, it is the case where starch or the like as the polysaccharide is maleated, and in the case of $NR_{11}$, it is the case where chitosan or chitin, and the like as the polysaccharide is maleated. The alkyl group of $R_{11}$ may be an alkyl group with 1 to 20 carbon atoms, 1 to 16 carbon atoms, 1 to 12 carbon atoms, 1 to 8 carbon atoms, or 1 to 4 carbon atoms, or a methyl group, and such an alkyl group may be linear, branched, or cyclic, and may also be optionally substituted with one or more substituents.

As described above, the self-cross-linking structure may be implemented by performing an esterification reaction between acidic polysaccharides, especially, polysaccharides having a carboxyl group. In the present application, by adjusting the reaction conditions, especially pH, in this self-cross-linking reaction, it is possible to provide a polymer material having balanced absorption physical properties.

As the acidic polysaccharides, polysaccharides having a carboxyl group on their own, such as CMC (carboxylmethyl cellulose), may be used, or polysaccharides in which a carboxyl group is introduced through a process such as maleation or carboxyalkylation may be used, where to achieve the above-mentioned substitution degree and for efficient self-cross-linking, polysaccharides into which a carboxyl group has been introduced through a denaturation process may be used.

The method of introducing a carboxyl group into a polysaccharide is not particularly limited. For example, to introduce a functional group such as Formula 4, a so-called maleation process may be performed. Such a process is a process of reacting a polysaccharide with an unsaturated dicarboxylic acid or its anhydride to replace the hydroxy group present in the unitary body of the polymer with the functional group, where an example of the dicarboxylic acid or its anhydride may be exemplified by maleic acid or maleic anhydride, and the like, but is not limited thereto, and salts of the maleic acid, and the like may also be applied. The method of performing the maleation process is known.

The carboxyalkylation process may be performed by reacting the polysaccharide with an alkanoic acid or haloalkanoic acid or a salt of the alkanoic acid or haloalkanoic acid. As is known, the alkanoic acid is an aliphatic acid derived from an alkane, and the haloalkano acid means one in which at least one of hydrogen atoms of the alkanoic acid is replaced with a halogen atom (e.g., chlorine, fluorine, or bromine, etc.). In this specification, the alkanoic acid, haloalkanoic acid, salt of alkanoic acid, and/or salt of haloalkanoic acid applied in the carboxyalkylation process may be referred to as a treatment agent.

As the alkano acid or haloalkano acid used as the treatment agent, an alkanoic acid or haloalkano acid with 1 to 20 carbon atoms, 1 to 16 carbon atoms, 1 to 12 carbon atoms, 1 to 8 carbon atoms, or 1 to 4 carbon atoms may be used, and acetic acid or chloroacetic acid may be typically used.

The salt of the haloalkanoic acid or alkanoic acid may be an alkali metal salt or alkali earth metal salt of the haloalkanoic acid or alkanoic acid having the same number of carbon atoms as above.

An acidic group (carboxyl group) may be introduced into the polysaccharide by reacting the treatment agent with the polysaccharide under appropriate conditions.

The process of introducing the acidic group may be performed according to a known method, and if necessary, an additional process may be performed or the conditions of the process may be adjusted for efficient progress of the carboxyalkylation process.

For example, the above process may be performed on a mixture in which the treating agent and a hydroxide are dispersed in a solvent. Here, as the solvent, for example, an aqueous solvent such as water may be used. Here, as the water, tap water, distilled water, deionized water, or purified water, and the like may be used. Here, as the hydroxide, ammonium hydroxide or a metal hydroxide may be used, and as the metal hydroxide, sodium hydroxide, potassium hydroxide, or lithium hydroxide, and the like may be used, without being limited thereto.

It is expected that in the reaction to the mixture, for example, a gelatinization reaction of polysaccharides first proceeds within the mixture, and subsequently introduction of carboxyl groups by the treatment agent proceeds.

The reaction may be performed, for example, while the torque of the reactor where the mixture exists after the gelatinization becomes a certain level. In this way, the introduction of a suitable carboxyl group proceeds.

For example, if the mixture is mixed in a mixer, such as an internal mixer, capable of mixing the components by rotation, the gelatinization proceeds, where heat energy is generated inside the reactor by the load caused by the gelatinization during a process that the gelatinization proceeds, whereby the torque and temperature increase. Typically, a section where the torque and temperature within the reactor increase to a certain level, and then remain constant occurs, where the time point when the section occurs is usually regarded as the time point when the gelatinization is completed. By maintaining the torque at an appropriate level at the gelatinization completion time point and/or the time point afterward to have a desired substitution degree, and the like, it is possible to obtain a polysaccharide capable of efficiently generating the self-cross-linking.

In one example, the lower limit of the torque in the reactor at the gelatinization completion time point and/or after the gelatinization completion time point may be 5 Nm, 5.5 Nm, 7 Nm, 7.5 Nm, or 8 Nm or so, and the upper limit thereof may be 20 Nm, 19 Nm, 18 Nm, 16 Nm, 15 Nm, 14 Nm, 13 Nm, 12 Nm, 11 Nm, 10 Nm, or 9 Nm or so. The torque may be more than or equal to, or more than any one of the above-described lower limits, or less than or equal to, or less than any one of the above-described upper limits, or within a range of less than or equal to, or less than any one of the above-described upper

limits while being more than or equal to, or more than any one of the above-described lower limits. By maintaining the torque in the above range, it is possible to prevent excessive evaporation of the solvent, and it is possible to maintain workability stably, while maintaining the substitution efficiency of the carboxyl group in the desired range.

To maintain the torque as above, the ratio of solvent in the mixture may be controlled. For example, the lower limit of the ratio of the solvent in the mixture may be 0.45 times, 0.5 times, 0.6 times, or 0.7 times or so the weight of the polysaccharide present in the mixture, and the upper limit thereof may be 0.75 times, 0.74 times, 0.73 times, or 0.72 times or so the weight of the polysaccharide present in the mixture. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. By maintaining the ratio of the solvent within the above range, it is possible to prevent excessive evaporation of the solvent, and it is possible to maintain the torque capable of stably maintaining workability, while maintaining the substitution efficiency of the carboxyl group in the desired range.

In the above reaction process, it may be appropriate to use substantially only the above-described aqueous solvent (e.g., water) as the solvent. Generally, as the solvent for carboxyalkylation, alcohol, ketone, 1,4-dioxane, dimethylformamide, or dimethyl sulfoxide, and the like may also be applied in addition to the aqueous solvent, but when such a solvent is applied, the reaction may not proceed as desired.

The mixture may contain substantially no solvent other than the aqueous solvent (e.g., water). At this time, the fact that it contains substantially no solvent may mean the case where the upper limit of the content of solvents other than the aqueous solvent (e.g., water) in the mixture is 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof is 0 wt% or so. The ratio may be less than any one of the above-described upper limits, or within a range of less than or equal to, or less than any one of the above-described upper limits while being more than or equal to, or more than any one of the above-described lower limits.

The ratios of the hydroxide and treating agent in the mixture may also be controlled.

For example, the lower limit of the ratio of the hydroxide in the mixture may be 0.5 equivalents, 0.6 equivalents, or 0.7 equivalents or so, and the upper limit thereof may be 1.5 equivalents, 1.15 equivalents, 1.1 equivalents, 1 equivalent, 0.9 equivalents, or 0.8 equivalents or so. The equivalent may be less than or equal to, or less than any one of the above-described upper limits, or within a range of less than or equal to, or less than any one of the above-described upper limits while being more than or equal to, or more than any one of the above-described lower limits. Here, the equivalent can be obtained by an equation A/B, wherein A is the mole number of the hydroxide present in the mixture, and B is a value calculated as C/162.14, where C is the weight (unit: g) of the polysaccharide in the mixture. Here, 162.14 is the molar mass (g/mol) of anhydroglucose unit. Typically, the polysaccharide contains the anhydroglucose unit or a derivative thereof, or a unit with a similar molar mass. Therefore, in the present application, if the used amount of polysaccharide for obtaining the above equivalent is representatively applied by the equation C/162.14, the reaction may proceed according to the purpose by specifying the equivalent according to such an applying manner in the above range. By maintaining the used amount of hydroxide in the above range, it is possible to stably maintain the reaction efficiency and workability while maintaining the substitution efficiency of carboxyl groups in the desired range, and it is possible to suppress unnecessary side reactions, and the like.

The lower limit of the ratio of the treatment agent in the mixture may be 0.5 equivalents, 0.6 equivalents, or 0.7 equivalents or so, and the upper limit thereof may be 1.5 equivalents, 1.15 equivalents, 1.1 equivalents, 1 equivalent, 0.9 equivalents, or 0.8 equivalents or so. The equivalent may be less than or equal to, or less than any one of the above-described upper limits, or within a range of less than or equal to, or less than any one of the above-described upper limits while being more than or equal to, or more than any one of the above-described lower limits. Here, the equivalent can be obtained by an equation D/B, wherein D is the mole number of the treatment agent present in the mixture, and B is the same as the equation for calculating the equivalent of the hydroxide. By maintaining the used amount of the treatment agent in the above range, it is possible to stably maintain the reaction efficiency and workability while maintaining the substitution efficiency of carboxyl groups in the desired range, and it is possible to suppress unnecessary side reactions, and the like.

The lower limit of the ratio (A/B) of the mole number (A) of the hydroxide and the mole number (D) of the treatment agent in the mixture may be 0.5, 0.6, 0.7, 0.8, 0.9, or 0.95 or so, and the upper limit thereof may be 1.5, 1.4, 1.3, 1.2, 1.1, or 1.05 or so. The ratio may be less than or equal to, or less than any one of the above-described upper limits, or within a range of less than or equal to, or less than any one of the above-described upper limits while being more than or equal to, or more than any one of the above-described lower limits. By maintaining the above ratio, it is possible to obtain a polysaccharide capable of stable self-cross-linking while maintaining the substitution efficiency of carboxyl groups in the desired range, it is possible to stably maintain the reaction efficiency and workability, and it is possible to suppress unnecessary side reactions, and the like.

In the above reaction, the mixture may be present in a predetermined ratio in a reactor where the reaction occurs. For example, the lower limit of the ratio of the volume of the mixture in the reactor based on the total volume of the reactor may be 70%, 75%, 76%, or 77% or so, and the upper limit thereof may be 95%, 94%, 93%, or 92% or so. The ratio may be

less than or equal to, or less than any one of the above-described upper limits, or within a range of less than or equal to, or less than any one of the above-described upper limits while being more than or equal to, or more than any one of the above-described lower limits. By maintaining the above ratio, it is possible to stably maintain torque in the reactor, it is possible to obtain a polysaccharide capable of stable self-cross-linking while maintaining the substitution efficiency of carboxyl groups in the desired range, it is possible to stably maintain the reaction efficiency and workability, and it is possible to suppress unnecessary side reactions, and the like.

In the above reaction, the temperature in the reactor at the gelatinization completion time point and/or after the time point may also be maintained at a certain level. For example, the lower limit of the temperature may be 90°C, 92°C, 94°C, or 96°C or so, and the upper limit thereof may be 110°C, 105°C, 100°C, 99°C, 98°C, or 97°C or so. The temperature may be less than or equal to, or less than any one of the above-described upper limits, or within a range of less than or equal to, or less than any one of the above-described upper limits while being more than or equal to, or more than any one of the above-described lower limits. By maintaining the above temperature, it is possible to stably maintain torque in the reactor, it is possible to obtain a polysaccharide capable of stable self-cross-linking while maintaining the substitution efficiency of carboxyl groups in the desired range, it is possible to stably maintain the reaction efficiency and workability, and it is possible to suppress unnecessary side reactions, and the like.

As described above, the carboxyalkylation may be performed using a reactor capable of mixing the mixture by rotation. The specific type of reactor is not particularly limited, and for example, an internal mixer such as a two-roll mixer, a Banbury mixer and an intermix mixer may be used. The torque and temperature may also be measured through sensors mounted on such a mixer.

The mixing time is not particularly limited, which may be controlled to a level where the desired gelatinization and carboxyalkylation may be achieved. For example, the lower limit of the mixing time may be 5 minutes, 7 minutes, 9 minutes, or 10 minutes or so, and the upper limit thereof may be 60 minutes, 55 minutes, 50 minutes, 45 minutes, 40 minutes, 35 minutes, 30 minutes, 25 minutes, 20 minutes, 15 minutes, 14 minutes, 13 minutes, 12 minutes, 11 minutes, or 10 minutes or so. The mixing time may be less than or equal to, or less than any one of the above-described upper limits, or within a range of less than or equal to, or less than any one of the above-described upper limits while being more than or equal to, or more than any one of the above-described lower limits.

The rotating speed of the mixing may be controlled to maintain the desired torque and/or temperature. The lower limit of the rotating speed may be, for example, 30 rpm, 35 rpm, 40 rpm, 45 rpm, or 50 rpm or so, and the upper limit thereof may be 100 rpm, 95 rpm, 90 rpm, 85 rpm, 80 rpm, 75 rpm, 70 rpm, 65 rpm, 60 rpm, 55 rpm, or 50 rpm or so. The rotating speed may be less than or equal to, or less than any one of the above-described upper limits, or within a range of less than or equal to, or less than any one of the above-described upper limits while being more than or equal to, or more than any one of the above-described lower limits.

Through the above process, the carboxyalkylation progresses, and it is possible to obtain the desired polysaccharide.

Immediately after introducing a carboxyl group into the polysaccharide through the reaction, it is also possible to perform the self-cross-linking process, and if necessary, after recovering the polysaccharide once, it is possible to perform the self-cross-linking process.

Here, the recovering of the polysaccharide may comprise a process of dissolving the reactant resulting from the reaction in water and precipitating it using an organic solvent such as alcohol, and in addition to this, various methods may be applied.

The polymer material may further comprise, together with the self-cross-linked polysaccharide component, a cross-linking agent binding with the polysaccharide component. Such a cross-linking agent may be introduced by reacting the self-cross-linked polysaccharide component with the cross-linking agent. That is, the polymer material of the present application may be provided in a state where two or more molecules of polysaccharide are cross-linked by the above-described self-cross-linking and then further cross-linked by applying the cross-linking agent. This further cross-linking is introduced for improving the gel strength of the self-cross-linked polysaccharide component and improving the absorption capacity under pressure (AUP) and/or saline flow conductivity. Typically, when the cross-linking agent is applied for this purpose, the absorption capacity under pressure of the polymer material is improved, but there is a problem that the absorption rate is lowered. However, in the present application, it is possible to provide a material exhibiting balanced absorption capacity even after application of the cross-linking agent through control of cross-linking conditions in the self-cross-linking step.

The additional cross-linking agent may also be introduced, for example, by subjecting the self-cross-linked polysaccharide component to a treatment such as grinding to powder it and reacting the surface of the relevant powder with the cross-linking agent. In this case, the polymer material may comprise the self-cross-linked polysaccharide component in a particle shape and the cross-linking agent bound to the surface of the particle. In such a case, the size of the particles is not particularly limited, and the particles may be controlled to an appropriate size depending on the applied use. Typically, the size of the particles may be in the range of approximately 100μm to 1,000μm.

As the cross-linking agent, a material having two or more functional groups capable of reacting with the functional

groups (hydroxy group, amino group, or carboxyl group, etc.) of the polysaccharide component may be used. The cross-linking agent may have 2 to 10, 2 to 9, 2 to 8, 2 to 7, 2 to 6, 2 to 5, 2 to 4, or 2 to 3, or 2 or 3 functional groups capable of reacting with the functional groups (hydroxy group, amino group, or carboxyl group, etc.) of the polysaccharide component.

As the type of the applicable cross-linking agent, one or more selected from the group consisting of a polyfunctional epoxy compound, an epoxy silane compound, an amino silane compound, epichlorohydrin, an acyl chloride, a carbonate, a diamine, a diol, carbon disulfide, phosphoryl chloride, divinyl benzene, an organic acid, and an organic acid anhydride may be used.

For performing effective cross-linking and securing desired physical properties, it may be advantageous to use a specific type of cross-linking agent.

In one example, as the cross-linking agent, an organic acid having two or more carboxyl groups, or an anhydride of the organic acid, or a carbonate-based compound may be applied.

The organic acid, anhydride of the organic acid, or organic compound may be composed of only carbon, oxygen, and hydrogen.

The type of the usable organic acid as the cross-linking agent is not particularly limited, but an organic acid with a molecular weight of about 90 g/mol to 300 g/mol, or about 100 g/mol to 250 g/mol may be used. The organic acid may have 2 to 10, 2 to 9, 2 to 8, 2 to 7, 2 to 6, 2 to 5, 2 to 4 or 2 to 3, or 2 or 3 carboxyl groups.

Such an organic acid may be exemplified by citric acid, succinic acid, pimelic acid, or adipic acid, and the like, but is not limited thereto.

The organic acid or its anhydride may be used as the cross-linking agent.

As the carbonate-based compound usable as the cross-linking agent, for example, an alkylene carbonate may be used, and for example, an alkylene carbonate in which the alkylene group has 1 to 20, 1 to 16, 1 to 12, 1 to 8, 1 to 4, 2 to 20, 2 to 16, 2 to 12, 2 to 8, 2 to 4 carbon atoms may be used.

The polymer material treated with the such a cross-linking agent can satisfy the desired absorption properties and biodegradability degree while exhibiting appropriate gel strength.

In the polymer material, the lower limit of the weight ratio of the cross-linking agent relative to 100 parts by weight of the self-cross-linked polysaccharide component may be 0.01 parts by weight, 0.05 parts by weight, 0.1 parts by weight, 0.5 parts by weight, 1 part by weight, 1.5 parts by weight, 2 parts by weight, 2.5 parts by weight, 3 parts by weight, 3.5 parts by weight, 4 parts by weight, 4.5 parts by weight, or 5 parts by weight or so, and the upper limit thereof may be 20 parts by weight, 18 parts by weight, 16 parts by weight, 14 parts by weight, 12 parts by weight, 10 parts by weight, 8 parts by weight, or 6 parts by weight or so. The ratio may be more than or equal to, or more than any one of the above-described lower limits, or less than or equal to, or less than any one of the above-described upper limits, or within a range of less than or equal to, or less than any one of the above-described upper limits while being more than or equal to, or more than any one of the above-described lower limits. Usually, disaccharides among polysaccharides exhibit molar masses in the above range.

Under such a ratio, the polymer material can satisfy the desired absorption properties and biodegradability degree while exhibiting appropriate gel strength.

The polymer material may comprise the polysaccharide component (polysaccharide component self-cross-linked and bound with the cross-linking agent) as described above, and may further comprise other components if necessary.

In one example, the lower limit of the ratio of the polysaccharide component (polysaccharide component self-cross-linked and bound with the cross-linking agent) in the polymeric material may be 55 wt%, 60 wt%, 65 wt%, 70 wt%, 75 wt%, 80 wt%, 85 wt%, 90 wt%, 92 wt%, 94 wt%, 96 wt%, or 98 wt% or so, and the upper limit thereof may be 100 wt%, 98 wt%, 96 wt%, 94 wt%, 92 wt%, or 90 wt% or so. The ratio may be less than any one of the above-described upper limits, or within a range of less than or equal to, or less than any one of the above-described upper limits while being more than or equal to, or more than any one of the above-described lower limits.

The ratio of the polysaccharide component in the polymer material is not particularly limited, but the higher the ratio, the greater the biodegradability of the polymer material. However, in the case of absorbent materials applying conventional polysaccharide components, if the ratio of polysaccharide components is excessively increased in consideration of biodegradability, there is a problem that the absorption capacity is reduced. However, in the present application, the desired absorption capacity can be stably achieved while maintaining the ratio of polysaccharide components at a high level.

The present application relates to a preparation method of such a polymer material.

The preparation method may comprise steps of self-cross-linking a polysaccharide to prepare the above-described self-cross-linked polysaccharide component, and reacting the prepared self-cross-linked polysaccharide component with the cross-linking agent.

As the self-cross-linking polysaccharide component, the above-described acidic polysaccharide (for example, the acidic polysaccharide having the degree of substitution) may be used.

The self-cross-linking of the polysaccharide may be performed under pH adjusted to a predetermined range. For example, the lower limit of pH at which the self-cross-linking is performed may be 9, 9.5, 10, or 10.5 or so, and the upper limit

thereof may be 11.35, 11, or 10.5 or so. The pH may be less than any one of the above-described upper limits, or more than or equal to, or more than any one of the above-described lower limits, or within a range of less than or equal to, or less than any one of the above-described upper limits while being more than or equal to, or more than any one of the above-described lower limits.

It is not clear why the desired resultant product can be obtained through self-cross-linking in the above pH range, but the pH affects the cross-linking rate of the polysaccharide or the cross-linking density of the self-cross-linked polysaccharide component, and such a result is strengthened in a reaction with the cross-linking agent to be described below, whereby it is expected that this is because a cross-linked structure capable of ultimately exerting balanced absorption capacity is realized.

The method of performing the self-cross-linking is not particularly limited, but for efficient self-cross-linking, it may be performed by a method of dispersing the polysaccharide in a solvent, and then maintaining the pH in the above range.

In the above process, an aqueous solvent, for example, water may be used as the solvent, and specifically, tap water, distilled water, deionized water, or purified water, and the like may be used. Upon the self-cross-linking, it may be appropriate to substantially use only the aqueous solvent (e.g., water) as the solvent. Therefore, the solvent used upon the self-cross-linking may not substantially contain other solvents rather than the aqueous solvent (e.g., water). At this time, the matter that other solvents are not substantially included may mean the case where the upper limit of the content of other solvents rather than the aqueous solvent (for example, water) in the solvent is 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof is 0 wt% or so. The ratio may be less than any one of the above-described upper limits, or within a range of less than or equal to, or less than any one of the above-described upper limits while being more than or equal to, or more than any one of the above-described lower limits.

The amount of the applied solvent n the above process may be an amount of 5 times to 15 times the weight of the applied polysaccharide. The dissolution of the polysaccharide into the solvent may be performed under room temperature and normal pressure conditions, but is not limited thereto.

The self-cross-linking may proceed by dissolving the polysaccharide in the solvent and maintaining the pH to the above-described range. If necessary, additional processes, such as a stirring process, capable of promoting the self-cross-linking may also be performed. The method of maintaining pH in the above range is not particularly limited, and if the pH in the above range is achieved by adding a polysaccharide, the self-cross-linking may proceed in that state, and if the desired pH is not achieved, the pH may be adjusted by adding an appropriate acid or base in consideration of the desired pH. At this time, for example, the hydroxide applied in the carboxyalkylation may be used as the base, and hydrochloric acid or sulfuric acid, and the like may be used as the acid, without being limited thereto.

The base may be mainly applied to maintain pH at a desired level. An example of the applicable base in this process includes NaOH, KOH, $K_2CO_3$, $Na_2CO_3$ and/or $NaHCO_3$, and the like, but an appropriate type may be selected from various other organic bases (e.g. amine series or ammonia series such as $NH_3$, $NH_4OH$, DIPEA (N,N-diisopropylethylamine), and TEA (triethylamine)) and/or inorganic bases ($Mg(OH)_2$, $Ca(OH)_2$, $Al(OH)_3$, $Ca_{10}(PO_4)_6(OH)_2$, $Li_2O$, $Na_2O$, $K_2O$, CaO, etc.).

In the above reaction process, a catalyst may be added as needed. For example, an ester catalyst promoting the reaction of carboxyl groups and hydroxy groups may be added. As such a catalyst, 4-methylaminopyridine, magnesium acetate, tetra-n-butyl titanate, lead acetate, sodium acetate, potassium acetate, antimony trioxide and/or N-methylimidazole, and the like may be used, without being limited thereto. The catalyst may be added in a catalytic amount and, for example, may be used in a ratio within a range of 0.1 mol to 5 mol relative to 1 mol of the polysaccharide applied to the reaction. The lower limit of the catalyst usage ratio may be 0.1 mol, 0.5 mol, 1 mol, or 2 mol or so, and the upper limit thereof may be 5 mol, 4.5 mol, 4 mol, or 3.5 mol or so. The ratio may be less than or equal to, or less than any one of the above-described upper limits, or within a range of less than or equal to, or less than any one of the above-described upper limits while being more than or equal to, or more than any one of the above-described lower limits.

The reaction may also be performed in the presence of a heat stabilizer, if necessary. As the applicable heat stabilizer, an organic or inorganic phosphorus compound such as phosphoric acid, an organic ester of phosphoric acid, phosphorous acid or an organic ester of phosphorous acid may be used, and for example, phosphoric acid, alkyl phosphate or aryl phosphate, and the like, which is commercially known as the heat stabilizer, may be used.

In addition, the reaction may also be performed in the presence of additives such as thickeners, plasticizers, preservation stabilizers, and/or antioxidants, if necessary.

The cross-linking reaction may be performed at a predetermined temperature. For example, the lower limit of the temperature at which the reaction proceeds may be 80°C, 85°C, 90°C, 95°C, 100°C, 105°C, 110°C, 115°C, or 120°C or so, and the upper limit thereof may be 300°C, 280°C, 260°C, 240°C, 220°C, 200°C, 180°C, 160°C, 140°C, 130°C, 125°C, or 125°C or so. The temperature may be less than or equal to, or less than any one of the above-described upper limits, or within a range of less than or equal to, or less than any one of the above-described upper limits while being more than or equal to, or more than any one of the above-described lower limits. Such a reaction temperature may be achieved by a method, such as hot air supply, infrared irradiation, microwave irradiation, or ultraviolet irradiation.

The time for the reaction to proceed is not particularly limited. For example, the lower limit of the reaction time may be 20 minutes, 40 minutes, 60 minutes, 80 minutes, 100 minutes, or 120 minutes or so, and the upper limit thereof may be 500 minutes, 480 minutes, 460 minutes, 440 minutes, 420 minutes, 400 minutes, 380 minutes, 360 minutes, 340 minutes, 320 minutes, 300 minutes, 280 minutes, 260 minutes, 240 minutes, 220 minutes, 200 minutes, or 180 minutes or so. The time may be less than or equal to, or less than any one of the above-described upper limits, or within a range of less than or equal to, or less than any one of the above-described upper limits while being more than or equal to, or more than any one of the above-described lower limits.

In one example, the process of obtaining the self-cross-linked polysaccharide component may be performed while evaporating or volatilizing the solvent at the temperature and time. The desired self-cross-linked polysaccharide component may be obtained by the above method.

The self-cross-linked polysaccharide component thus obtained may react with the cross-linking agent in a subsequent reaction.

After the self-cross-linking process, the polysaccharide component may also react with the cross-linking agent as such, and may react with the cross-linking agent after additional treatment to increase the cross-linking efficiency, if necessary. For example, after the self-cross-linking process, the polysaccharide component may be granulated, and then reacted with the cross-linking agent. For example, the polysaccharide component may be granulated through an appropriate grinding and/or classification process, and the granulated polysaccharide component may react with the cross-linking agent.

In this case, the process may comprise steps of granulating the self-cross-linked polysaccharide component; and reacting the surface of the granulated self-cross-linked polysaccharide component with the cross-linking agent.

The method of reacting the polysaccharide component and the cross-linking agent is not particularly limited. For example, a method of contacting the polysaccharide component with a cross-linking agent and reacting them in an appropriate reaction medium such as a solvent, and the like may be applied. When the granulated polysaccharide component reacts with the cross-linking agent, a method of spraying the cross-linking agent or a cross-linking liquid containing the cross-linking agent onto the surface of the granulated polysaccharide component to bring into contact with it, and performing the reaction may also be used.

In this case, the above-described cross-linking agent may be applied as the cross-linking agent.

The reaction with the cross-linking agent may also be carried out in the presence of additives such as catalysts, thickeners, plasticizers, preservation stabilizers, and/or antioxidants, if necessary.

The cross-linking reaction may be performed at a predetermined temperature. For example, the lower limit of the temperature at which the reaction proceeds may be 80°C, 85°C, 90°C, 95°C, 100°C, 105°C, 110°C, 115°C, or 120°C or so, and the upper limit thereof may be 300°C, 280°C, 260°C, 240°C, 220°C, 200°C, 180°C, 160°C, 140°C, 130°C, 125°C, or 125°C or so. The temperature may be less than or equal to, or less than any one of the above-described upper limits, or within a range of less than or equal to, or less than any one of the above-described upper limits while being more than or equal to, or more than any one of the above-described lower limits. Such a reaction temperature may be achieved by a method, such as hot air supply, infrared irradiation, microwave irradiation, or ultraviolet irradiation.

The time for the reaction to proceed is not particularly limited. For example, the lower limit of the reaction time may be 20 minutes, 40 minutes, 60 minutes, 80 minutes, 100 minutes, or 120 minutes or so, and the upper limit thereof may be 500 minutes, 480 minutes, 460 minutes, 440 minutes, 420 minutes, 400 minutes, 380 minutes, 360 minutes, 340 minutes, 320 minutes, 300 minutes, 280 minutes, 260 minutes, 240 minutes, 220 minutes, 200 minutes, or 180 minutes or so. The time may be less than or equal to, or less than any one of the above-described upper limits, or within a range of less than or equal to, or less than any one of the above-described upper limits while being more than or equal to, or more than any one of the above-described lower limits.

Following the reaction with such a cross-linking agent, the desired polymer material may be obtained through additional processes (for example, grinding/classification processes, etc.), if necessary.

The polymer material is made of a biodegradable material and exhibits balanced absorbency, so that it may be used for various applications.

For example, the polymer material may be used as sanitary products such as diapers or sanitary napkins, or absorbent materials applied to other applications requiring absorption. If necessary, further cross-linking, surface treatment, or physical grinding processes may also be performed on the polymer material to increase the efficiency for use as the sanitary products or absorbent materials.

Therefore, the present application relates to an absorbent material or sanitary product (e.g., diapers, sanitary napkins, etc.) comprising the polymer material.

The specific method of forming the absorbent material or sanitary product by applying the polymer material is not particularly limited, and for example, the method of forming the absorbent material or sanitary product by applying the existing SAP may be used in the same manner.

**Advantageous Effects**

The present application can provide a polymer material, a preparation method therefor, and a use thereof. The present application can provide a polymer material made of a material with biodegradability and exhibiting balanced absorption properties. The present application can also provide a preparation method for such a polymer material, and a use thereof.

**Mode for Invention**

Hereinafter, the present application will be described in detail below through examples and comparative examples, but the scope of the present application is not limited by the following examples.

**1. Evaluation of centrifuge retention capacity (CRC)**

Centrifuge retention capacity (CRC) was measured according to EDANA (European Disposables and Nonwovens Association) WSP 241.3. About 0.2 g ($W_0$) of the obtained polymer material was placed in a non-woven bag, sealed, and then submerged in a physiological saline solution. As the physiological saline solution, an aqueous NaCl solution with a concentration of 0.9 wt% was used. The state was maintained for 30 minutes or so, water was removed from the bag for 3 minutes under a condition of 250 G using a centrifuge, and the mass (g, $W_2$) of the bag was measured.

The same operation was performed on the same non-woven bag containing no polymer material, and the mass (g, $W_1$) was measured.

The CRC (g/g) was calculated by substituting the measurement results into Equation A below.

The evaluation was conducted under constant temperature and humidity conditions (23 ± 1°C, relative humidity: 50 ± 10%).

[Equation A]

$$CRC\ (g/g) = \{[W_2(g) - W_1(g)]/W_0(g)\} - 1$$

2. Evaluation of absorption capacity under pressure (AUP)

Absorption capacity under pressure (AUP, 0.7 psi) of a polymer material was measured according to the standard of EDANA (European Disposables and Nonwovens Association) WSP 242.3. A 400-mesh stainless steel wire net was mounted on the bottom of a plastic cylinder with an inner diameter of 60 mm or so. Under conditions of a temperature of 23 ± 2°C and a relative humidity of 50%, about 0.90 g ($W_0$) of the polymer material was uniformly sprayed on the wire net, and a piston capable of further uniformly imparting a load of about 0.7 psi was installed thereon to manufacture a measuring device. As the piston, a piston with an outer diameter slightly smaller than 60 mm was used, which was installed so that it could move up and down without forming a gap with the inner wall of the cylinder. The weight (unit: g) ($W_3$) of the measuring device was measured.

A glass filter with a diameter of 90 mm or so and a thickness of 5 mm or so was placed on the inside of a petro dish with a diameter of about 150 mm, and a physiological saline solution (NaCl aqueous solution with a concentration of 0.9 wt%) was applied to be at the same level as the upper surface of the glass filter. One sheet of filter paper with a diameter of 90 mm or so was placed thereon. The measuring device was placed on the filter paper and the physiological saline solution was absorbed for 1 hour under a load of 0.7 psi. After 1 hour, the measuring device was lifted, and the weight $W_4$ (g) was measured.

The absorption capacity under pressure (AUP) (g/g) was calculated by substituting the respective weights as measured into Equation B below.

[Equation B]

$$AUP\ (g/g) = [W_4(g) - W_3(g)]/W_0(g)$$

3. **Measurement of biodegradability degree**

A biodegradability degree was measured in the manner specified in ISO 14855-1 (2005) standard. The above standard is a method of measuring an aerobic biodegradability degree of plastic materials under a composting condition,

which is a method that the biodegradability degree of a polymer material is calculated by quantifying the amount of carbon dioxide emitted by microorganisms metabolizing the relevant material. As the polymer material was applied to the composting condition according to the standard, the biodegradability degree was measured for 6 months, and the biodegradability degree was obtained as the ratio of the theoretical carbon dioxide generation amount and the actual carbon dioxide generation amount of the material. Here, the theoretical carbon dioxide generation amount and biodegradability degree are obtained according to the following equations C and D, respectively.

[Equation C]

$$\text{Theoretical carbon dioxide generation amount (ThCO2, g/container)} = \text{MTOT} \times \text{CTOT} \times (44/12)$$

In Equation C, MTOT is the amount (g) of total dry solid content in the test material (polymer material) added to the compost at the start of the measurement, and CTOT means the ratio (g/g) of organic carbon contained in the total dry solid content of the test material.

[Equation D]

$$\text{Biodegradability degree (\%)} = [\{(\text{CO2})\text{T}-(\text{CO2})\text{B}\}/\text{ThCO2}] \times 100$$

In Equation D, (CO2)T is the accumulated amount (g/container) of carbon dioxide generated from the composting container containing the test material, (CO2)B is the average (g/container) of carbon dioxide accumulated amounts generated from the inoculum source container, and ThCO2 is the theoretical carbon dioxide generation amount confirmed in Equation C above.

**4. Evaluation of vortex absorption time**

About 50 mL of a 0.9 wt% NaCl aqueous solution (physiological saline) was added to a beaker, and a cylindrical stirring bar (diameter: about 6 mm, length: about 30 mm) was placed in the aqueous solution. About 0.1 g of each polymer material prepared in Examples or Comparative Examples was added to the beaker while rotating the stirring bar on a stirring plate at a rotation speed of about 600 rpm. If the above state is maintained, vortexes of the physiological saline solution are observed on the surface of the polymer material while the polymer material absorbs the physiological saline, but when absorption by the polymer material is completed, no vortex is observed. The time from the addition time point of the polymer material to the time point when no vortex was observed was measured and defined as an absorption rate.

**Example 1.**

A self-cross-linked polysaccharide component was prepared by self-cross-linking CMC (carboxymethyl cellulose) as a lignocellulosic polysaccharide. 20 g of CMC (Carboxymethyl cellulose) was dissolved in 800 mL of distilled water, spread thinly on a tray, and then dried in an oven at 40°C. NaOH aqueous solution of about 0.5N was added to the mixture of CMC (carboxymethyl cellulose) and distilled water to adjust the pH to 10.5 or so. After the drying, CMC (carboxymethyl cellulose) was heated at 120°C for 3 hours to prepare a self-cross-linked polysaccharide component, and the polysaccharide component was pulverized and classified to obtain a material having a particle size of about 300 μm to 600 μm or so.

Surface cross-linking was performed on the obtained material. The surface cross-linking was performed using a surface cross-linking solution prepared by dissolving 0.072 g of propylene carbonate together with 0.018 g of $AlCl_3$ in a solution of 0.627 g of acetone and 0.4 g of water. 3.6 g of the material pulverized and classified to have a particle size of about 300 μm to 600 μm or so was placed on an aluminum dish, and the surface cross-linking solution was uniformly sprayed. After mixing the cross-linking agent until it was sufficiently dissolved, it was heated at 120°C for 30 minutes to obtain a polymer material comprising a self-cross-linked polysaccharide component and the cross-linking agent bound to the component. The obtained material can be further pulverized and classified as needed.

**Example 2.**

A polymer material was prepared in the same manner as in Example 1, except that as the cross-linking agent, succinic acid was applied instead of propylene carbonate. The surface cross-linking solution was prepared by mixing

0.072 g of succinic acid, 1.26 g of acetone, and 0.4 g of water. The cross-linking was performed by spraying the cross-linking solution evenly on the surface of the self-cross-linked particulate polysaccharide component, and then maintaining it at a temperature of 120°C or so for 1 hour.

**Example 3.**

A polymer material was obtained in the same manner as in Example 2, except that the pH was controlled to 11.06 by adjusting the added amount of NaOH upon self-cross-linking.

**Comparative Example 1.**

A polymer material was obtained in the same manner as in Example 2, except that the pH was controlled to 11.37 by adjusting the added amount of NaOH upon self-cross-linking.

**Comparative Example 2.**

A polymer material was obtained in the same manner as in Example 2, except that the pH was controlled to 7 by adjusting the added amount of NaOH upon self-cross-linking.

**Comparative Example 3.**

A polymer material was obtained in the same manner as in Example 2, except that the pH was controlled to 9 by adjusting the added amount of NaOH upon self-cross-linking.

**Comparative Example 4.**

A polymer material was obtained in the same manner as in Example 2, except that the pH was controlled to 6.56 by adding HCl upon self-cross-linking.

**Comparative Example 5.**

A polymer material was obtained in the same manner as in Example 2, except that the pH was controlled to 4.41 by adding HCl upon self-cross-linking.

**Comparative Example 6.**

A polymer material was obtained in the same manner as in Example 2, except that the pH was controlled to 5.24 by adding HCl upon self-cross-linking.

The physical property measurement results for the polymer materials of Examples and Comparative Examples are shown in Table 1 below. In the case of Comparative Examples 5 and 6, the centrifuge retention capacity (CRC) and absorption capacity under pressure (AUP) among the absorption characteristics were too low, so that the vortex absorption time could not be measured.

[Table 1]

| | CRC (g/g) | AUP (g/g) | Vortex absorption time (sec) |
|---|---|---|---|
| Example 1 | 44.8 | 10.3 | 69 |
| Example 2 | 35.12 | 11.75 | 102 |
| Example 3 | 35.92 | 10.36 | 103 |
| Comparative Example 1 | 12.41 | 8.72 | 201 |
| Comparative Example 2 | 26.05 | 10.75 | 216 |
| Comparative Example 3 | 25.87 | 12.99 | 226 |
| Comparative Example 4 | 17.26 | 12.65 | 420 |
| Comparative Example 5 | 1.47 | 2.93 | - |

(continued)

| | CRC (g/g) | AUP (g/g) | Vortex absorption time (sec) |
|---|---|---|---|
| Comparative Example 6 | 4.41 | 6.18 | - |

**Claims**

1. A polymer material comprising:

   a self-cross-linked polysaccharide component; and a cross-linking agent bound with the self-cross-linked polysaccharide component, and having
   a vortex absorption time of 150 seconds or less for 0.9 wt% NaCl aqueous solution.

2. The polymer material according to claim 1, wherein the self-cross-linked polysaccharide component is in a particle shape and the cross-linking agent is bound to the surface of the particle shape.

3. The polymer material according to claim 1, having an absorption capacity under pressure at 0.7 psi of 10 g/g or more according to EDANA (European Disposables and Nonwovens Association) method WSP 242.3.

4. The polymer material according to claim 1, having a centrifuge retention capacity of 20 g/g or more according to EDANA (European Disposables and Nonwovens Association) method WSP 241.3.

5. The polymer material according to claim 1, having a biodegradability degree of 50% or more.

6. The polymer material according to claim 1, wherein the polysaccharide is an acidic polysaccharide having a substitution degree in a range of 0.1 to 2.5.

7. The polymer material according to claim 1, wherein the self-cross-linked polysaccharide component comprises polymer chains containing monosaccharide units linked by glycosidic bonds, and bonds of Formula 1 below linking the polymer chains:

   [Formula 1]

   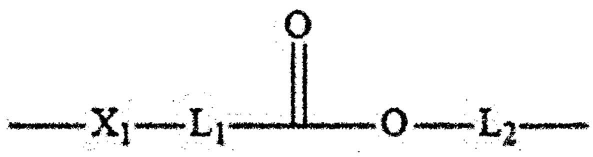

   wherein, $X_1$ is an oxygen atom or $NR_{11}$, where $R_{11}$ is a hydrogen atom, an alkyl group or an alkylcarbonyl group, and $L_1$ is an alkylene group, an alkylidene group, or a bond of Formula 2 below, and $L_2$ is represented by a single bond or $-(CH_2)-O-$.

   [Formula 2]

   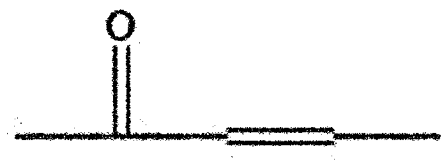

8. The polymer material according to claim 7, wherein the monosaccharide unit has a ring structure including carbon atoms and oxygen atoms as ring constituent atoms, and the bond of Formula 1 is directly connected to the carbon atom of the ring structure, or is connected via a methylene group.

**9.** The polymer material according to claim 1, wherein the polysaccharide component contains a unit represented by Formula 3 below:

[Formula 3]

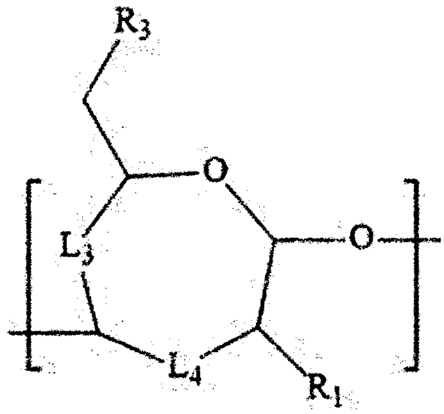

wherein, $R_1$ is a hydroxy group, an amino group, -$L_5$-C(=O)-OH, -$L_5$-C(=O)-O-, or a functional group of Formula 4 below, $R_3$ is a hydroxy group, -$L_5$-C(=O )-OH, -$L_5$-C(=O)-O- or a functional group of Formula 4 below, and one of $L_3$ and $L_4$ is a single bond, the other is $CHR_2$, where $R_2$ is a hydroxy group, -$L_5$-C ( =O)-OH, -$L_5$-C(=O)-O- or a functional group of Formula 4 below, and $L_5$ is an alkylene group or an alkylidene group, but any one of $R_1$ to $R_3$ is the oxygen atom (excluding the oxygen atom present in the carbonyl group) of the bond of Formula 1 above.

[Formula 4]

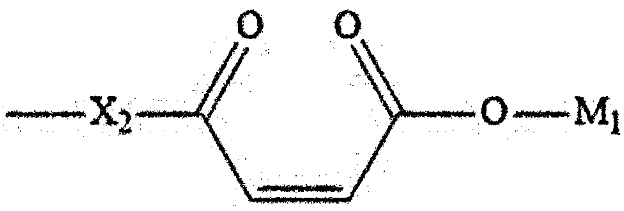

wherein, $X_2$ is an oxygen atom or $NR_{11}$, where $R_{11}$ is a hydrogen atom, an alkyl group or an alkylcarbonyl group, $M_1$ is hydrogen or a metal, and when $M_1$ is the metal, the bond of O-$M_1$ above is an ionic bond.

**10.** The polymer material according to claim 1, wherein the cross-linking agent is one or more selected from the group consisting of a polyfunctional epoxy compound, an epoxy silane compound, an amino silane compound, epichlorohydrin, an acyl chloride, a carbonate, a diamine, a diol, carbon disulfide, phosphoryl chloride, divinyl benzene, an organic acid, and an organic acid anhydride.

**11.** The polymer material according to claim 1, wherein the cross-linking agent is an organic acid having two or more carboxyl groups, or an anhydride of the organic acid.

**12.** The polymer material according to claim 1, wherein the cross-linking agent is an alkylene carbonate.

**13.** The polymer material according to claim 1, comprising 0.01 to 20 parts by weight of the cross-linking agent relative to 100 parts by weight of the self-cross-linked polysaccharide component.

**14.** A method of preparing a polymer material comprising steps of:

cross-linking a polysaccharide under conditions where pH is more than 9, and 11.35 or less to prepare a self-cross-linked polysaccharide component; and
reacting the self-cross-linked polysaccharide component with a cross-linking agent.

**15.** The method of preparing a polymer material according to claim 14, comprising steps of: granulating the self-cross-linked polysaccharide component; and reacting the surface of the granulated self-cross-linked polysaccharide component with a cross-linking agent.

**16.** An absorbent material comprising the polymer material of any one of claims 1 to 13.

**17.** A sanitary article comprising the polymer material of any one of claims 1 to 13.

## INTERNATIONAL SEARCH REPORT

International application No.
**PCT/KR2024/000880**

### A. CLASSIFICATION OF SUBJECT MATTER

**C08J 3/24**(2006.01)i; **C08J 3/12**(2006.01)i; **C08B 15/00**(2006.01)i; **C08L 1/28**(2006.01)i; **C08K 5/00**(2006.01)i; **C08K 5/092**(2006.01)i; **C08K 5/109**(2006.01)i; **A61L 15/22**(2006.01)i

According to International Patent Classification (IPC) or to both national classification and IPC

### B. FIELDS SEARCHED

Minimum documentation searched (classification system followed by classification symbols)

C08J 3/24(2006.01); A61K 31/715(2006.01); C07H 5/06(2006.01); C08B 31/10(2006.01); C08B 37/00(2006.01); C08J 3/28(2006.01); C08L 5/00(2006.01); C08L 89/00(2006.01); G01N 21/3577(2014.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 자가-가교(self-crosslinking), 다당류 (polysaccharide), 전분(starch), 가교제(crosslinking agent)

### C. DOCUMENTS CONSIDERED TO BE RELEVANT

| Category* | Citation of document, with indication, where appropriate, of the relevant passages | Relevant to claim No. |
|---|---|---|
| X | KR 10-1242010 B1 (ARCHER-DANIELS-MIDLAND COMPANY) 13 March 2013 (2013-03-13) See claims 1, 7 and 10-12; paragraphs [0012], [0023]-[0027], [0112], [0119]-[0124], [0134], [0176], [0177] and [0200]-[0202]; and table 3. | 1-17 |
| A | KR 10-2001-0105311 A (SCA HYGIENE PRODUCTS ZEIST B.V.) 28 November 2001 (2001-11-28) See abstract; claims 1 and 12-14; and page 6. | 1-17 |
| A | JP 6055466 B2 (THE UNIVERSITY OF TOKYO) 27 December 2016 (2016-12-27) See claims 1-21. | 1-17 |
| A | JP 2008-111027 A (JAPAN ATOMIC ENERGY AGENCY) 15 May 2008 (2008-05-15) See claims 1-10. | 1-17 |
| A | US 2008-0300218 A1 (ABE, Y. et al.) 04 December 2008 (2008-12-04) See entire document. | 1-17 |

☐ Further documents are listed in the continuation of Box C.   ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 April 2024** | **29 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/000880**

| Patent document cited in search report | Publication date (day/month/year) | Patent family member(s) | Publication date (day/month/year) |
|---|---|---|---|
| KR 10-1242010 B1 | 13 March 2013 | AU 2007-302586 A1 | 03 April 2008 |
| | | AU 2007-302586 B2 | 27 June 2013 |
| | | BR PI0717171 A2 | 15 October 2013 |
| | | BR PI0717171 B1 | 17 October 2023 |
| | | CA 2664392 A1 | 03 April 2008 |
| | | CN 101541836 A | 23 September 2009 |
| | | CN 101541836 B | 08 April 2015 |
| | | CN 104774275 A | 15 July 2015 |
| | | EP 2066699 A1 | 10 June 2009 |
| | | EP 2066699 B1 | 12 February 2020 |
| | | JP 2010-504414 A | 12 February 2010 |
| | | JP 2013-253262 A | 19 December 2013 |
| | | JP 5765884 B2 | 19 August 2015 |
| | | JP 5947768 B2 | 06 July 2016 |
| | | KR 10-1329658 B1 | 14 November 2013 |
| | | MX 2009003252 A | 02 November 2009 |
| | | MX 339603 B | 31 May 2016 |
| | | US 2008-0177057 A1 | 24 July 2008 |
| | | US 2013-0296548 A1 | 07 November 2013 |
| | | US 8461129 B2 | 11 June 2013 |
| | | WO 2008-037082 A1 | 03 April 2008 |
| KR 10-2001-0105311 A | 28 November 2001 | AU 2000-18975 A1 | 03 July 2000 |
| | | AU 2000-18975 B2 | 27 November 2003 |
| | | BR 9916235 A | 04 September 2001 |
| | | CA 2356849 A1 | 22 June 2000 |
| | | EP 1140229 A1 | 10 October 2001 |
| | | EP 1140229 B1 | 06 October 2010 |
| | | JP 2002-532573 A | 02 October 2002 |
| | | MX PA01006098 A | 27 March 2002 |
| | | NZ 512254 A | 28 November 2003 |
| | | PL 348821 A1 | 17 June 2002 |
| | | TN SN99243 A1 | 31 December 2001 |
| | | US 2004-0236016 A1 | 25 November 2004 |
| | | US 6765042 B1 | 20 July 2004 |
| | | WO 00-35504 A1 | 22 June 2000 |
| | | ZA 200104559 B | 04 June 2002 |
| JP 6055466 B2 | 27 December 2016 | WO 2013-176239 A1 | 28 November 2013 |
| JP 2008-111027 A | 15 May 2008 | None | |
| US 2008-0300218 A1 | 04 December 2008 | EP 1595892 A1 | 16 November 2005 |
| | | JP 4638817 B2 | 23 February 2011 |
| | | US 2006-0178339 A1 | 10 August 2006 |
| | | US 7485719 B2 | 03 February 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230008025